# EUROPEAN PATENT SPECIFICATION

(11) **EP 1 178 316 B1**
(45) Date of publication and mention of the grant of the patent: **12.01.2005**
(21) Application number: 01850136.1
(22) Date of filing: 03.08.2001
(51) Int. Cl.: G01N 33/543

(54) **Medical Kit and method for the determination of a drug**
Medizinische Kit und Methode zur Bestimmung eines Arzneimittels
Trousse et méthode médicales pour déterminer un médicament

(30) Priority: 04.08.2000 SE 0002820
(43) Date of publication of application: 06.02.2002
(73) Proprietor: The Jordanian Pharmaceutical Manufacturing and Medical Equipment Co.Ltd., Jo-Naor 11710 (JO)
(72) Inventor: Badwan, Adnan, Jo-Amman (JO); Saleem, Mohammed, Jo-Amman (JO); Jehanli, Ahmed, Mitcham, Surrey CR4 4BL (GB)
(74) Representative: Winkler, Andreas, Dr.

(56) References cited:
- EP-A- 0 101 912
- DE-A- 19 722 815
- US-A- 5 238 652
- AHMED M.T. JEHANLI ET AL: "Determination of captopril in human blood by an enzyme-linked immunosorbent assay" J. PHARM. PHARMACOL., vol. 48, 1996, pages 914-917, XP001029006
- KHALID MATALKA ET AL: "Enzyme linked immunosorbent assay for determination of amilodipine in plasma" JOURNAL OF CLINICAL LABORATORY ANALYSIS, vol. 15, no. 1, 2001, pages 47-53, XP001029223

## Description

### Field of the Invention

The present invention relates to a medical kit for qualitative or quantitative determination of a drug in a biological fluid as well as a method therefor.

### Background of the Invention

The determination of an effective therapeutic dose of a drug is often difficult, and this is mainly due to a great individual variation with respect to drug absorption and metabolism. An individual variation is also commonly seen due to the effect of *e.g.* food, body weight and possible interaction with other drugs present. Although this individual variation is not of major concern for some drugs, *e*.*g*. aspirin, it is nevertheless of importance for most other drugs. Indeed, with very potent drugs, particularly those used in treatment of cancer and heart diseases, a non-optimal dose may yield highly detrimental, even fatal, results. Consequently, a plethora of different methods have been developed in order to provide a qualitative or quantitative determination of drugs in biological fluids.

Many of the present methods used for the determination of drugs in biological fluids are based on rather complex procedures, such as those requiring HPLC, GC or GC/MS equipment. Evidently, such procedures are often inconvenient for both individual and rapid drug monitoring directly at the point-of-care, such as at home or basically at any location in- or outside a healthcare facility. As is well known, various medical kits which allow drug monitoring at the point-of care have therefore been developed. Among the public, the most well known of these kits is probably the pregnancy test Clearblue®.

Said medical kits are usually based on some kind of immunoassay. Furthermore, they normally require lateral flow or diffusion through a carrier or membrane in order to function properly (see *e.g.* EP 833 159). However, the main problem associated with medical kits requiring significant lateral flow, or diffusion, is that non-specific binding and interaction between the present reagents, or analytes, and the carrier is often observed. Such non-specific binding and interaction reduces the reliability of the analysis.

Moreover, there are ongoing demands in the art to provide a medical kit which allows the following:
rapid determination of drug levels in a biological fluid, where rapid typically means less than 60 minutes from biological fluid sampling to result;
simple and reliable use thereof, also for individuals not having any particular medical knowledge;
low energy input and/or no required controlled heating when used;
low or negligible temperature dependence;
storage stability; and
manufacturing at low costs, thus making the medical kit attractive for use also in third-world countries.

### Disclosure of the Invention

According to the present invention, there is now provided a novel medical kit which overcomes the problem with non-specific binding and interaction, thus providing a highly reliable method for qualitative or quantitative determination of a drug in a biological fluid. Also the aforementioned demands in the art are well met by the present invention. More specifically, the present invention relates to a medical kit for qualitative or quantitative determination of a drug in a biological fluid, said medical kit comprising:
*i)* a first part coated with a drug conjugate; and
*ii)* a second part which contains a labelled anti-drug antibody and is adapted for receiving said biological fluid, wherein said labelled anti-drug antibody is labelled with gold material and/or latex particles. Preferably, said drug conjugate is a conjugate between said drug and a protein, peptide, polyamine, ceramide, alkyl chain or carbohydrate. Most preferably, said protein is selected from rabbit serum albumin (RSA), bovine serum albumin, ovalbumin, gamma globulin and thyro globulin.

In an embodiment of the invention the latex particles are coloured. Coloured latex particles are commercially available from a number of sources such as Polymer Laboratories Ltd., Essex Road, Shropshire, England and Bangs Laboratories, US. Examples of latex particles are "Dyed polymer microspheres" from Bangs Laboratories Inc., US, catalogue No. DS03B, 0,5-0,9 µm diameter. These microsphere particles have an intense blue colour. They bind proteins by adsorption. Polymer Laboratories Ltd., Shropshire, England also provides latex particles for antibody labelling, for example PL-Latex Plain HiDype™ Blue 400nm, Catalogue No. 6004-412.

In another embodiment of the invention the gold material used as labelling material is colloidal gold. The colloidal gold may comprise metallic gold as well as gold ions. The novel use of gold-labelled and/or coloured latex particles labelled antibodies for detection of analytes in a solid phase/liquid setting according to the invention have many advantages over the prior art.
Firstly, the use of colloidal gold instead of enzyme as label makes this embodiment substantially unaffected by normal temperature fluctuations, which is especially advantageous from a reproducability, storage as well as user aspect. Secondly, this embodiment provides an extremely rapid analysis (5-15 minutes from sampling to result), since the colour development requires no additional step involving a substrate for a labelling enzyme. Indeed, the use of a gold material such as colloidal gold is particularly preferred according to the present invention.

Preferably, said drug is selected from antihypertensive, antiviral, antimicrobial, antifungal, antiinflammatory, antitumor and psychopharmaceutical agents, including corticosteroids, mono- to oligosaccharides, vitamins, provitamins and hormones.

It is particularly preferred that said drug has a molar weight in the range of from 50 to 6000 g/mol, preferably from 100 to 1000 g/mol.

Typically, said drug is selected from lisinopril, amilodipine, captopril, enalapril, enalaprilat, ketotifen, sildenafil and fluoxetine.

In the present medical kit, said second part is preferably a container which either contains a solution of said labelled anti-drug antibody or has its interior at least partially coated therewith. Typically, said container is tube-shaped.

As an example, said first part typically has the shape of a stick, strip, paddle or plate. The shape of a Nunc-Immuno™ Stick (*vide infra*) is particularly preferred.

As for the preferred material of said first part, its surface is typically of a material selected from polystyrene, polypropylene, nitrocellulose material chemically treated for maximum binding.

The material below the surface may be of either the same or a different material as that of the surface. The surface of said first part may thus be either completely integrated with the underlying material, *i.e*. said first part is made of a single type of material, or is a layer applied on virtually any kind of useful material. Preferably, the surface of the first part is that used for the surface of a Nunc-Immuno™ Stick. In a preferred embodiment, said first part is a Nunc-Immuno™ Stick, as exemplified hereinbelow. By using the Nunc-Immuno™ Stick as the first part (i) mentioned above for the solid phase immunoassay according to the invention surprisingly good results are obtained. It is specially preferred due to the advantage of a rapid near-patient medical kit for monitoring drug levels in biological fluids of patients.

Moreover, the present invention also relates to a method for qualitative or quantitative determination of a drug in a biological fluid, wherein a first part coated with a drug conjugate, *i.e.* a first part *i)* as defined above, and a labelled anti-drug antibody, wherein said labelled anti-drug antibody is labelled with gold material and/or latex particles, are utilised.

In accordance with said method, said first part is preferably placed inside a second part which contains a labelled anti-drug antibody and is adapted for receiving said biological fluid, *i.e.* a second part *ii)* as defined above, wherein said second part contains said biological fluid, and wherein said first part is removed from said second part after a predetermined period of time.

Said predetermined period of time is typically in the range of from 5 to 60 minutes.

According to said method, said first part is subsequently, *i.e*. after the aforementioned steps have been performed, developed in order to bring about a potential colour change indicating the level of said drug in said biological fluid.

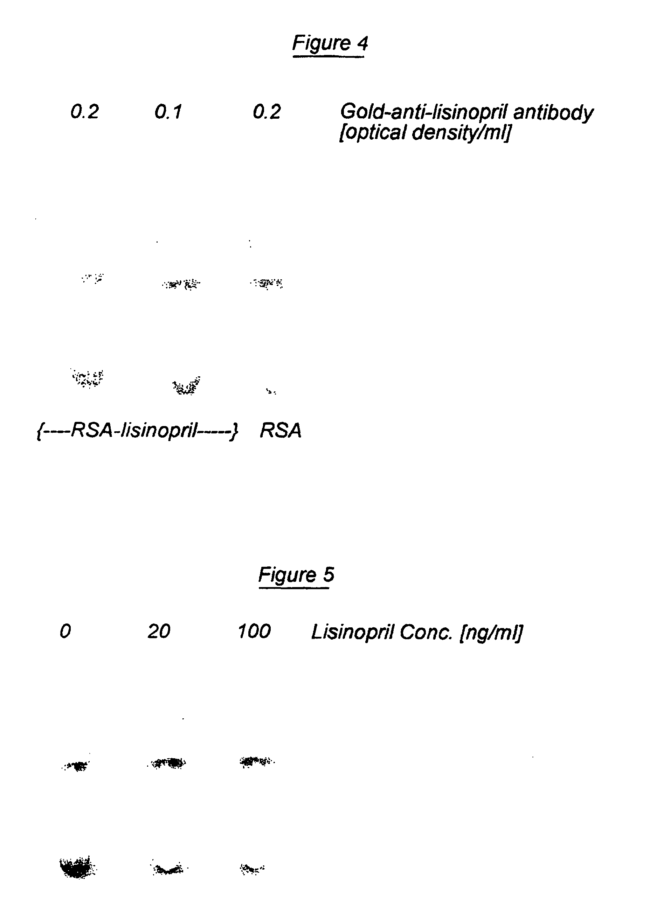

When using colloidal gold when labelling the anti-drug antibody in connection with the present invention extremely good results are obtained, ie dark brown-red colour, see figure 4. Gold can also be regarded as a comparable cheap substance to use for labelling, especially in comparison with certain expensive purified enzymes often used as labelling material.

The kit according to the invention has the advantage that it can be used by an unskilled person for self-monitoring of a drug in a biological sample, ie urine, blood or saliva, easily without the aid of any additional instrumentation.

Usually, said method in its entirety is performed at ambient temperature. Typically, said biological fluid is blood, serum, urine or saliva.

As used herein, the expression "drug" comprises any substance which may yield or relate to a physiological response or condition, particularly when administered to a living organism. Thus, for the purposes of the present invention, also metabolites (active or non-active), prodrugs and antigens are considered as drugs.

. The present invention is further illustrated by the following non-limiting examples together with the accompanying figures.

### Description of the figures

Fig. 1 depicts a standard curve for an HRP labelled anti-lisinopril antibody present at a concentration of 25 ng/ml. The detected levels of lisinopril (pg/ml) were monitored at a wavelength of 450 nm.

Fig. 2 depicts titration of HRP labelled anti-lisinopril antibody against sticks coated with either an RSA-lisinopril conjugate or RSA only.

Fig. 3 depicts the ability of the present medical kit to both detect and distinguish between different levels of lisinopril in human whole blood by utilising said first part coated with an RSA-lisinopril conjugate. An HRP labelled anti-lisinopril antibody was used for detection.

Fig. 4 depicts titration of gold-labelled (Au³⁺) rabbit anti-lisinopril antibody against sticks coated with either an RSA-lisinopril conjugate or RSA only.

Fig. 5 depicts detection of different levels of lisinopril in human whole blood by utilising said first part coated with an RSA-lisinopril conjugate and said second part containing gold-labelled rabbit anti-lisinopril antibody for detection.

### Examples

### Materials and procedures:

Drug conjugates were prepared essentially in accordance with the methodology presented in "Bioconjugate Techniques", Ed. Hermanson, G., Academic Press, USA, 1996. The preparation of the anti-drug antibodies was essentially performed by following the procedures set forth in "Synthetic polypeptides as antigens", Eds. van Regen-mortel, M.H.V., Briand, J.P., Muller, S. and Plaue, S., Elsevier, Holland, 1988. For preparation of anti-drug antibodies in general, see also "Practice and theory of enzyme immunoassays", Tijssen, P., Elsevier Publishing, Holland, 1985, chapters 5 and 12. The entire teachings of all the publications referred to above are incorporated herein by reference.

### Principle of the present invention when using the Nunc immunostick as the first part (i) mentioned above:

In this assay the analytes present in the biological fluid competes with the standard analyte which is immobilised on the sticks for binding with the enzyme-labelled , preferably gold-labelled, anti-analyte antibody. The procedure will be described with reference to lisinopril. The Nunc paddles are coated with the protein-lisinopril conjugate. This binds strongly by non-covalent interaction. Excess protein-drug conjugate is washed away and any free binding sites on the sticks are blocked by incubation with a protein- and detergent-containing buffer e.g., casein and Tween-20. After blocking, the sticks are washed in water, dried and can be stored under dry condition until further use. In the test, the sticks are placed in a tube containing a dilution of the enzyme-labelled, preferably gold-labelled, anti-lisinopril antibody and a few drops of the biological fluid being tested. After 5-10 minutes incubation to allow for the antigen-antibody reaction, the sticks are washed in tap water to remove excess labelled antibody. If the biological fluid does not have the drug, then most of the labelled antibody will bind to the drug immobilised on the sticks. This binding will reveal itself directly, in the case of gold-labelled antibody, as the gold-antibody has intense reddish colour. In the case of enzyme-labelled antibody, the binding of the antibody is revealed by the addition of a substrate (TMB in the case of horseradish peroxidase). The enzyme-labelled antibody, which is now bound to the drug immobilised on the stick, will convert the soluble substrate into an insoluble blue coloured product that will be deposited on the surface of the stick. The sticks are rinsed in water to remove excess substrate solution and dried. If the biological fluid contains the drug, then this will compete with the immobilised drug for binding with the antibody conjugate. This means that less antibody conjugate will bind to the drug immobilised on the sticks. Hence, the sticks will be coloured less intensely. The more free drug present in the biological fluid the less the colour intensity would be. In practice, an antibody conjugate dilution is selected that will give intense colour at or below the cut off concentration and no colour or faint colour at the "positive" concentration, e.g., for lisinopril the cut off chosen is 50 ng/ml. At this concentration or below sufficient antibody conjugate will bind to give intense colour, while at 100 ng/ml the colour intensity is faint or absent.

Alternatively, an indirect standard immunoassay procedure may be used instead of directly labelling the anti-drug antibody (e.g., rabbit anti-lisinopril antibody). In this embodiment one can use a labelled second antibody, e.g., enzyme-labelled, or gold-labelled goat anti-rabbit IgG antibody. In this case, the assay will be performed as before but with the addition of an extra step. Hence, the coated strips will be incubated with the anti-drug antiserum (rabbit anti-lisinopril) for 5-10 minutes, followed by washing in water for 10 seconds, incubation with labelled goat anti-rabbit IgG for another 5-10 minutes, washing and reading for gold-labelled antibody directly or after the addition of the substrate in the case of enzyme labelled antibody.

Yet another variation to the indirect test is to label the anti-drug antibody with biotin and using gold-labelled or enzyme labelled avidin or streptavidin in place of the second antibody.

### Preparation of Rabbit Serum Albumin-Lisinopril conjugates by 2-step glutaraldehyde method:

1. 10 mg of rabbit serum albumin is dissolved gently in 1 ml of PBS (phosphate buffered saline: 0.02M Na₂HPO₄/NaH₂PO₄ buffer, pH 7.4 containing 0.15M NaCl).
2.1 ml of freshly prepared 1 % (w/v) glutaraldehyde is added to the protein dropwise while stirring. Leave stirring for 2 hours at room temperature.
3. Excess glutaraldehyde is removed by gel filtration in PBS, using either Sephadex G-25F or Sephadex G-50M. A column with at least 20 ml of gel (recommend 1.5 x 20cm) will be needed. Monitor protein peak at 280 nm.
4. Fractions containing the protein are pooled and lisinopril solution in PBS is added. A molar ratio of lisinopril: RSA of 200: 1 is used. Incubation while mixing for 24 hours at room temperature.
5. Dialyse vs. PBS for 48 hours at 4°C.
6. Optical density is measured at 280nm. Conjugate concentration using the extinction coefficients for a 1 mg/ml protein concentration: 0.67 at 280 nm is worked out.
Sodium azide is added to 0.1 % (w/v) and aliquots are stored at -20°C.

### Preparation of IgG-Horseradish peroxidase conjugates (HPR-IgG) by 2-step glutaraldehyde method:

1. 10 mg of HRP is dissolved gently in 1 ml of PBS (phosphate buffered saline: Na₂HPO₄/NaH₂PO₄ buffer, pH 7.4 containing 0.15M NaCl).
2.1 ml of freshly prepared 1 % (w/v) glutaraldehyde is added to the enzyme dropwise while stirring. Leave stirring for 2 hours at room temperature.
3. Excess glutaraldehyde is removed by gel filtration in PBS, using either Sephadex G-25F or Sephadex G-50M. A column with at least 20 ml of gel (recommend 1.5 x 10 cm) will be needed. Monitor enzyme peak at 403 nm.
4. Fractions containing the enzyme are pooled and antibody solution in PBS is added. A molar ratio of HRP: IgG of 4-6: 1 is used. The proteins are incubated while mixing for 24 hours at room temperature or 4°C if IgG is labile.
5. Dialyse vs. PBS for 48 hours at 4°C.
6. O.D. is measured at 403 nm and 278 nm. HRP and IgG concentrations in the conjugate using the extinction coefficients for a 1 mg/ml protein concentration are worked out:
   HRP₄₀₃ₙₘ = 2.2, HRP₂₇₈ₙₘ = 0.75, IgG₂₇₈ₙₘ = 1.4.
7. BSA is added to 10 mg/ml, Tween-20 to 0.05 % (w/v) and a preservative: 0.005 % thiomersal or still better 0.02 % (w/v) bromo-nitro-dioxane (BND). Aliquot are stored at -20°C.

### Note:

HRP-drug conjugates are made following the same procedure, using drug: HRP molar ratio of 5:1.

Rabbit anti-lisinopril antibody was gold-labelled by British Biocell International, Cardiff, Wales, Britain.

### Sensitivity evaluation:

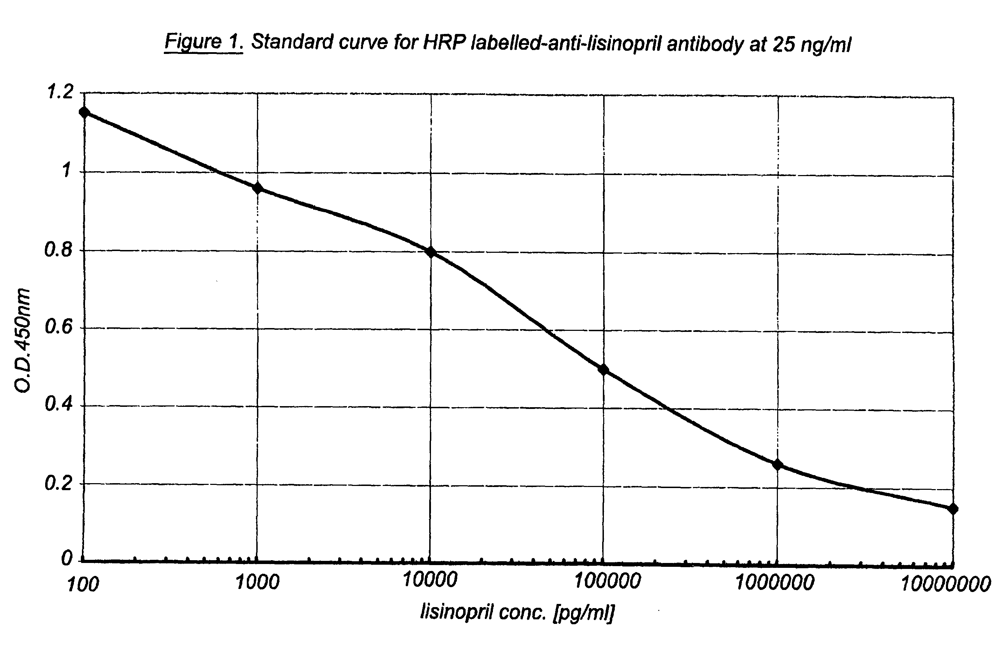

The interior walls of conventional microtitre wells were coated with an RSA-lisinopril conjugate by adding a solution of said conjugate (1 µg/ml; 0.1 ml) to said wells followed by incubation for 1 h at 37°C. After washing with wash buffer (10 mM sodium phosphate buffered saline, pH 7.4 containing 0.05 % sodium azide and 0.05 % Tween-20, PBS-T), 250 µl/well, 3 times, the wells were incubated with varying amounts of lisinopril in human whole blood (25 µl) in the presence of HRP labelled anti-lisinopril antibody (25 ng/ml in wash buffer, 100 µl/well) for 30 min at room temperature. After washing with wash buffer the wells were incubated with the substrate, tetramethylbenzidine solution, 100 µl/well for 30 min at room temperature. Colour development was quenched by addition of 2 M sulphuric acid, 100 µl/well and the colour intensity was measured at a wavelength of 450 nm by use of a Dynex microtitre plate reader. The results are depicted in Fig. 1. Since a lisinopril level as low as 100 pg/ml could be detected, this experiment verified that the sensitivity of the assay system of the present invention is very high.

### Evaluation of possible non-specific binding:

Sticks coated with RSA-lisinopril conjugate were prepared by submerging a Nunc-Immuno™ Stick (manufactured by Life Technologies, UK) into a solution of RSA-lisinopril conjugate (5 µg/ml) in carbonate buffer (pH 9.5), followed by incubation for 1 h at 37°C. Phosphate buffered saline (PBS; pH 7.4) may optionally be used as solvent, and the incubation may alternatively be performed for 24 h at room temperature. After the incubation, the stick was washed in tap water and blocked in PBS containing 0.2% casein, 0.05% Tween-20 and 0.05% NaN₃ (preservative) for 1 h at room temperature. Then it was rinsed once in tap water, dried at room temperature and stored in a dry environment until further use thereof.

The above describes a generally applicable coating procedure, and sticks coated with RSA were prepared in complete accordance therewith.

Moreover, some details of the coating procedure deserve additional comments. Firstly, a direct coupling of the drug to a stick would not yield a useful product, since the fairly low molecular weight of the drug would make it nearly impossible for an antibody to directly bind to the immobilised drug. For this reason, sticks were instead coated with a drug conjugate, preferably a protein-drug conjugate such as RSA-lisinopril. Conjugates between a drug and a polyamine or carbohydrate are equally useful. Secondly, it was also found that *i*) coating with a high amount of conjugate, and *ii)* a high drug/protein ratio in the conjugate were absolutely crucial for attaining high sensitivity. Typically, the concentration of the conjugate in the above coating solution should be in the range of 1-5 µg/ml, whereas a useful drug/protein ratio in the conjugate should be from 10:1 to 200:1.

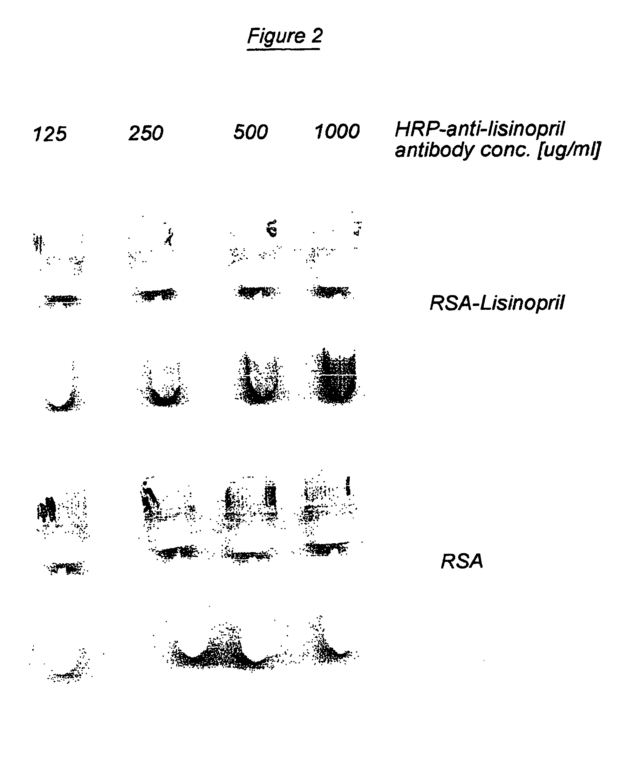

The sticks prepared as above were then inserted into tubes containing 10% (v/v) human whole blood in phosphate buffered saline (0.5 ml), containing 0.05% Tween-20, 0.2% casein and 10 mM EDTA (pH 7.4), as well as various concentrations of HRP labelled anti-lisinopril antibody. After incubation for 5 min at room temperature, each stick was removed, rinsed in tap water for 10 seconds and then inserted into tubes containing tetramethylbenzidine substrate for 5 minutes, after which it was rinsed as before, dried and photographed. The results are depicted in Fig. 2, and they confirm that non-specific antibody binding to the sticks is insignificant in comparison with the very high degree of specific antibody binding. A major prerequisite for the versatility of the present invention is thereby provided. For practical purposes, the most suitable colour intensity was obtained when the concentration of the HRP labelled anti-lisinopril antibody was 250 ng/ml.

### Detection of lisinopril in human whole blood:

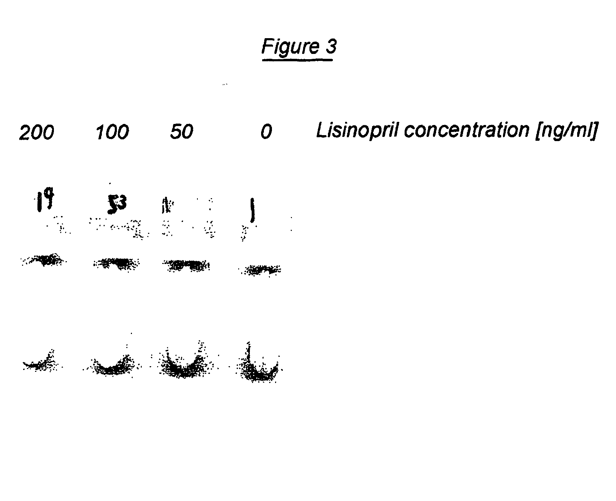

Sticks coated with RSA-lisinopril conjugate as above were submerged into a mixture (500 µl) of HRP labelled anti-lisinopril antibody (250 ng/ml; 400 µl; in phosphate buffered saline (0.5 ml), containing 0.05% Tween-20, 0.2% casein and 10 mM EDTA (pH 7.4)), and human whole blood (100 µl) containing various concentrations of lisinopril. After incubation for 5 min, the sticks were removed, rinsed in tunning tap water and developed with tetramethylbenzidine as above. The results are shown is Fig. 3, and they clearly illustrate a marked colour intensity difference between the various evaluated lisinopril concentrations. Note in particular the clear difference in colour intensity between the blood samples containing 50 ng/ml and 100 ng/ml, respectively, of lisinopril. Since these concentrations cover the effective therapeutic range of this drug, the results *supra* should be of considerable medical relevance.

### Evaluating the binding specificity of gold-labelled rabbit anti-lisinopril antibody:

Colloidal gold is made up of clusters of gold ions. Colloidal gold is not a molecule of defined structure, but suspended particles with some of the properties of metallic gold. In solution, colloidal gold adopts a negative charge, thought to arise from gold iodine chloride (AuICl₂) which make up the surface of the particle. Because of this negative charge, colloidal gold has an affinity for many proteins, which tend to have a positive or neutral charge at physiological pH. This allows adsorption of antibodies to colloidal gold particles. The colloidal gold particles themselves have intense red colour. Hence, when the gold-labelled antibody is concentrated at the site of the corresponding antigen, you get intense brown-reddish colour whose intensity will obviously depend on the number of antibody molecules bound.

Colloidal gold labelled antibodies are commercially available to certain antigens. For example, colloidal gold labelled anti-drugs of abuse antibodies can be bought from many companies such as British Biocell International (BBI), Cardiff, Wales, United Kingdom. In the present invention the labelling is performed by BBI using their propriety techniques. This is important as each antibody may require certain means for labelling. It is however possible to perform the gold labelling directly by using a suspension of colloidal gold that can be purchased from BBI.

Sticks coated with either RSA-lisinopril conjugate or RSA only were prepared as described above. They were then incubated for 20 min at room temperature with gold-labelled rabbit anti-lisinopril antibody (200 µl; 0.2 or 0.1 optical density units/ml; in phosphate buffered saline (0.5 ml), containing 0.05% Tween-20, 0.2% casein and 10 mM EDTA (pH 7.4),) in the presence of human whole blood (50 µl). After the incubation the stick was removed and rinsed with tap water for 10 seconds, whereby potential colour (red) development was immediate. The results are shown in Fig. 4, and they evidence a very high binding specificity also for this system. The solution of gold-labelled rabbit anti-lisinopril antibody having an optical density of 0.2/ml was most preferred for further use.

### Detection of lisinopril in human whole blood by utilising gold-labelled antibody:

Sticks coated with RSA-lisinopril conjugate as above were submerged into mixtures of gold-labelled rabbit anti-lisinopril antibody (200 µl; 0.2 optical density units/ml; in phosphate buffered saline (0.5 ml), containing 0.05% Tween-20, 0.2% casein and 10 mM EDTA (pH 7.4),) and human whole blood (50 µl), the latter containing lisinopril at the concentrations 0, 20 and 100 ng/ml, respectively. After incubation for 15 min, the sticks were removed and rinsed in running tap water for about 10 seconds, providing immediate colour development. The results are shown in Fig. 5, and they clearly illustrate a dinstinguishable colour intensity difference between the evaluated lisinopril concentrations.

The characteristics of the present embodiment which utilises gold-labelled anti-drug antibody require some additional comments. Firstly, the use of gold instead of enzyme as label makes this embodiment substantially unaffected by normal temperature fluctuations, which is especially advantageous from a reproducability, storage as well as user aspect. Secondly, this embodiment provides an extremely rapid analysis (5-15 minutes from sampling to result), since the colour development requires no additional step involving a substrate for a labelling enzyme. Indeed, this embodiment is particularly preferred.

According to preliminary experiments, the optional use of anti-drug antibody labelled with latex particles (*e.g.* carboxy activated beads, Bangs Laboratories Inc., U.S.A.) yields a product having properties nearly identical to those of the gold-labelled anti-drug antibody system disclosed above.

To summarise, the present medical kit is typically used in practise as follows:

A solution of about 200-500 µl of the labelled anti-drug antibody is added to a second part, normally a tube, followed by addition of about 50-100 µl of the tested biological fluid, normally blood. After incubation for 1-3 minutes, a first part, *i.e*. a coated stick prepared as above, is placed inside the tube and incubated for 5-10 minutes. The stick is then removed, rinsed in tap water for 10 seconds and developed. The concentration of the solution of the labelled anti-drug antibody should preferably be selected so that test samples containing a drug level below the effective therapeutic dose yield a fairly intensely stained stick, whereas test samples containing a drug level within the effective therapeutic dose should yield only a weakly stained, or even unstained, stick. The entire procedure is normally performed at ambient temperature and requires less than 60 minutes from blood sampling to result. Hence, it should be evident that the concept of the present invention provides a significant contribution to the art.

## Claims

1. A medical kit for qualitative or quantitative determination of a drug in a biological fluid, said medical kit comprising:
*i)* a first part coated with a drug conjugate; and
*ii)* a second part which contains a labelled anti-drug antibody and is adapted for receiving said biological fluid, wherein said labelled anti-drug antibody is labelled with gold material and/or latex particles.

2. A medical kit according to claim 1, wherein said drug conjugate is a conjugate between said drug and a protein, peptide, polyamine, ceramide, alkyl chain or carbohydrate.

3. A medical kit according to claim 2, wherein said protein is selected from rabbit serum albumin, bovine serum albumin, ovalbumin, gamma globulin and thyro globulin.

4. A medical kit according to any one of claims 1-3, wherein said latex particles are coloured.

5. A medical kit according to any one of claims 1-4, wherein said gold material is colloidal gold.

6. A medical kit according to any one of claims 1-5, wherein said drug is selected from antihypertensive, antiviral, antimicrobial, antifungal, antiinflammatory, antitumor and psychopharmaceutical agents, including corticosteroids, mono- to oligosaccharides, vitamins, provitamins and hormones.

7. A medical kit according to any one of claims 1-6, wherein said drug has a molar weight in the range of from 50 to 6000 g/mol, preferably from 100 to 1000 g/mol.

8. A medical kit according to claim 7, wherein said drug is selected from lisinopril, amilodipine, captopril, enalapril, enalaprilat, ketotifen, sildenafil and fluoxetine.

9. A medical kit according to any one of claims 1-8, wherein said second part is a container which either contains a solution of said labelled anti-drug antibody or has its interior at least partially coated therewith.

10. A medical kit according to any one of claims 1-9, wherein said first part has the shape of a stick, strip, paddle or plate.

11. A medical kit according to claim 10, wherein said first part has the shape of a Nunc-Immuno™ Stick.

12. A medical kit according to any one of claims 1-11, wherein said first part has a surface of a material selected from polystyrene, polypropylene, nitrocellulose material chemically treated for maximum binding.

13. A medical kit according to claim 12, wherein said material is that used for the surface of a Nunc-Immuno^{TM} Stick.

14. A medical kit according to claim 9, wherein said container is tube-shaped.

15. A medical kit according to any one of claims 1-14, wherein said biological fluid is blood, serum or urine.

16. A method for qualitative or quantitative determination of a drug in a sample of a biological fluid, wherein a first part *i*) as defined in any one of claims 1-13 is placed inside a second part *ii)* as defined in any one of claims 1-14, wherein said second part contains said biological fluid, wherein said first part is removed from said second part after a predetermined period of time.

17. A method according to claim 17, wherein said predetermined period of time is in the range of from 5 to 60 minutes.

18. A method according to any one of claims 16-18, wherein said first part is subsequently developed in order to bring about a potential colour change indicating the level of said drug in said biological fluid.

19. A method according to any one of claims 16-19, wherein the method in its entirety is performed at ambient temperature.

20. A method according to any one of claims 16-20, wherein said biological fluid is blood, serum, urine or saliva.

## Patentansprüche

1. Ein medizinisches Kit zur qualitativen oder quantitativen Bestimmung eines Arzneimittels in einer biologischen Flüssigkeit, wobei das medizinische Kit folgendes umfasst.
i) einen ersten Teil, der mit einem Arzneimittelkonjugat beschichtet ist; und
ii) einen zweiten Teil, der einen markierten Antiarzneimittelantikörper enthält und dahingehend angepasst ist, um die biologische Flüssigkeit aufzunehmen, wobei der markierte Antiarzneimittelantikörper mit Goldmaterial und/oder Latexteilchen markiert ist.

2. Ein medizinisches Kit nach Anspruch 1, worin das Arzneimittelkonjugat ein Konjugat zwischen dem Arzneimittel und einem Protein, Peptid, Polyamin, Ceramid, einer Alkylkette oder einem Kohlenhydrat ist.

3. Ein medizinisches Kit nach Anspruch 2, worin das Protein aus Kaninchenserum-albumin, Rinderserumalbumin, Ovalbumin, Gammaglobulin. und Thyroglobulin ausgewählt ist.

4. Ein medizinisches Kit nach einem der Ansprüche 1 - 3, worin die Latexteilchen gefärbt sind.

5. Ein medizinisches Kit nach einem der Ansprüche 1 - 4, worin das Goldmaterial kolloidales Gold ist.

6. Ein medizinisches Kit nach einem der Ansprüche 1 - 5, worin das Arzneimittel aus blutdrucksenkenden, antiviralen, antimikrobiellen, antimykotischen, entzündungshemmenden, Antitumor- und psychopharmazeutischen Mitteln, einschließlich Corticosteroiden, Mono- bis Oligosacchariden, Vitaminen, Provitaminen und Hormonen ausgewählt ist.

7. Ein medizinisches Kit nach einem der Ansprüche 1 - 6, worin das Arzneimittel ein Molargewicht in dem Bereich von 50 bis 6000 g/mol, vorzugsweise 100 bis 1000 g/mol, aufweist.

8. Ein medizinisches Kit nach Anspruch 7, worin das Arzneimittel aus Lisinopril, Amilodipin, Captopril, Enalapril, Enalaprilat, Ketotifen, Sildenafil und Fluoxetin ausgewählt ist.

9. Ein medizinisches Kit nach einem der Ansprüche 1 - 8, worin der zweite Teil ein Behälter ist, der entweder eine Lösung des markierten Antiarzneimittelantikörpers enthält oder dessen Innenseite wenigstens teilweise damit ausgekleidet ist.

10. Ein medizinisches Kit nach einem der Ansprüche 1 - 9, worin der erste Teil die Form eines Stabes, Streifens, einer Schaufel oder Platte hat.

11. Ein medizinisches Kit nach Anspruch 10, worin der erste Teil die Form eines Nunc-Immuno™- Stabes hat.

12. Ein medizinisches Kit nach einem der Ansprüche 1 - 11, worin der erste Teil eine Oberfläche eines Materials, das aus Polystyrol, Polypropylen, Nitrocellulosematerial, das für eine maximale Bindung chemisch behandelt ist, ausgewählt ist, aufweist.

13. Ein medizinisches Kit nach Anspruch 12, worin das Material dasjenige ist, welches für die Oberfläche eines Nunc-Immuno^{TM} - Stabes verwendet wird.

14. Ein medizinisches Kit nach Anspruch 9, worin der Behälter schlauchförmig ist.

15. Ein medizinisches Kit nach einem der Ansprüche 1 - 14, worin die biologische Flüssigkeit Blut, Serum oder Urin ist.

16. Ein Verfahren zur qualitativen oder quantitativen Bestimmung eines Arzneimittels in einer Probe einer biologischen Flüssigkeit, worin ein erster Teil i), wie er in einem der Ansprüche 1 - 13 definiert wird, in einen zweiten Teil ii), wie er in einem der Ansprüche 1 - 14 definiert wird, platziert wird, wobei der zweite Teil die biologische Flüssigkeit enthält, wobei der erste Teil aus dem zweiten Teil nach einem vorbestimmten Zeitraum entfernt wird.

17. Ein Verfahren nach Anspruch 17, worin der vorbestimmte Zeitraum in dem Bereich von 5 bis 60 Minuten liegt.

18. Ein Verfahren nach einem der Ansprüche 16 - 18, worin der erste Teil anschließend entwickelt wird, um einen potentiellen Farbwechsel zu bewirken, der die Menge des Arzneimittels in der biologischen Flüssigkeit anzeigt.

19. Ein Verfahren nach einem der Ansprüche 16 - 19, worin das Verfahren in seiner Gesamtheit bei Raumtemperatur ausgeführt wird.

20. Ein Verfahren nach einem der Ansprüche 16 - 20, worin die biologische Flüssigkeit Blut, Serum, Urin oder Speichel ist.

## Revendications

1. Trousse médicale pour la détermination qualitative ou quantitative d'un médicament dans un fluide biologique, ladite trousse médicale comprenant :
*i)* une première partie revêtue d'un conjugué du médicament ; et
*ii)* une seconde partie qui contient un anticorps anti-médicament marqué et est adaptée pour recevoir ledit fluide biologique, dans laquelle ledit anticorps anti-médicament marqué est marqué avec un composant d'or et/ou des particules de latex.

2. Trousse médicale selon la revendication 1, dans laquelle ledit conjugué de médicament est un conjugué entre ledit médicament et une protéine, un peptide, une polyamine, un céramide, une chaîne alkyle ou un glucide.

3. Trousse médicale selon la revendication 2, dans laquelle ladite protéine est choisie parmi la sérumalbumine de lapin, la sérumalbumine de boeuf, l'ovalbumine, la gammaglobuline et la thyroglobuline.

4. Trousse médicale selon l'une quelconque des revendications 1 à 3, dans laquelle lesdites particules de latex sont colorées.

5. Trousse médicale selon l'une quelconque des revendications 1 à 4, dans laquelle ledit composant d'or est un or colloidal.

6. Trousse médicale selon l'une quelconque des revendications 1 à 5, dans laquelle ledit médicament est choisi parmi les agents anti-hypertenseurs, antiviraux, antimicrobiens, antifongiques, anti-inflammatoires, antitumoraux et psychopharmaceutiques, comprenant les corticostéroïdes, les saccharides (mono- à oligo-), les vitamines, les provitamines et les hormones.

7. Trousse médicale selon l'une quelconque des revendications 1 à 6, dans laquelle ledit médicament a une masse molaire dans la plage de 50 à 6000 g/mole, de préférence, de 100 à 1000 g/mole.

8. Trousse médicale selon la revendication 7, dans laquelle ledit médicament est choisi parmi le lisinopril, l'amilodipine, le captopril, l'énalapril, l'énalaprilat, le kétotifène, le sildénafil et la fluoxétine.

9. Trousse médicale selon l'une quelconque des revendications 1 à 8, dans laquelle ladite seconde partie est un récipient, soit qui contient une solution dudit anticorps anti-médicament marqué, soit dont l'intérieur est au moins partiellement revêtu avec celle-ci.

10. Trousse médicale selon l'une quelconque des revendications 1 à 9, dans laquelle ladite première partie a la forme d'un stick, d'une bandelette, d'une ailette ou d'une plaque.

11. Trousse médicale selon la revendication 10, dans laquelle ladite première partie a la forme d'un Stick Nunc-Immuno™.

12. Trousse médicale selon l'une quelconque des revendications 1 à 11, dans laquelle ladite première partie a une surface à base d'un matériau choisi parmi le polystyrène, le polypropylène, une nitrocellulose traitée chimiquement pour une liaison maximale.

13. Trousse médicale selon la revendication 12, dans laquelle ledit matériau est celui utilisé pour la surface d'un Stick Nunc-Immuno™.

14. Trousse médicale selon la revendication 9, dans laquelle ledit récipient a la forme d'un tube.

15. Trousse médicale selon l'une quelconque des revendications 1 à 14, dans laquelle ledit fluide biologique est le sang, le sérum ou l'urine.

16. Méthode pour la détermination qualitative ou quantitative d'un médicament dans un échantillon de fluide biologique, dans laquelle une première partie *i)* telle que définie dans l'une quelconque des revendications 1 à 13 est placée à l'intérieur d'une seconde partie *ii)* telle que définie dans l'une quelconque des revendications 1 à 14, dans laquelle ladite seconde partie contient ledit fluide biologique, dans laquelle ladite première partie est retirée de ladite seconde partie après un laps de temps prédéterminé.

17. Méthode selon la revendication 16, dans laquelle ledit laps de temps prédéterminé est dans la plage de 5 à 60 minutes.

18. Méthode selon l'une quelconque des revendications 16 à 17, dans laquelle ladite première partie est ensuite révélée de manière à induire un changement de couleur potentiel indiquant le niveau dudit médicament dans ledit fluide biologique.

19. Méthode selon l'une quelconque des revendications 16 à 18, dans laquelle la méthode dans sa totalité est mise en oeuvre à la température ambiante.

20. Méthode selon l'une quelconque des revendications 16 à 19, dans laquelle ledit fluide biologique est le sang, le sérum, l'urine ou la salive.
